# EUROPEAN PATENT APPLICATION

(11) **EP 1 661 621 A1**
(43) Date of publication of application: **31.05.2006**
(21) Application number: 05017703.9
(22) Date of filing: 13.08.2005
(51) Int. Cl.: B01J 31/40, B01J 31/22, B01J 31/02, C07C 29/40, C07C 29/86, C07C 33/30, C07B 53/00, C07B 63/00, B01D 15/32

(54) **Asymmetric fluorous catalytic systems with recovery of catalyst ligand by solid phase extraction**

(30) Priority: 24.09.2004 EP 04022878
(71) Applicant: Merck Patent GmbH, 64293 Darmstadt (DE)
(72) Inventor: Dr. Vaughan-Spickers, Julian, Southampton SO52 9NE (GB); Prof.Hope,Eric George, Warwichshire CV23 0GA (GB); Dr.Stuart, Alison, Leicester LE7 4XU (GB); West, Andrew, Leicester LE9 1RL (GB)

(57) **Abstract**

The present invention relates to a process for the recovery and the reuse of catalytic systems involving 1,1'-Bi-2-Naphthol (BINOL) and titanium tetraisopropoxide from reaction mixtures comprising organotin species. A further object of the invention is a method for catalyzing an asymmetric reaction in high conversion rate and enantioselectivity.

## Description

### Technical Field

The present invention relates to a process for the recovery and the reuse of catalytic systems involving 1,1'-Bi-2-Naphthol (BINOL) and titanium tetraisopropoxide. A further object of the invention is a new method for catalyzing an asymmetric reaction in high conversion rate and enantioselectivity.

### Background Art

Asymmetric catalytic systems involving 1,1'-Bi-2-Naphthol (BINOL) and titanium tetraisopropoxide are well documented.

For example, Kitamoto et al. [*Tetrahedron Lett.,* 36, **1995,** 1861] described a chiral 1,1'-bi-2-naphthol(BINOL)-derived titanium complex, prepared via complete hydrolysis of the complex formed in situ by mixing (i-PrO)₄Ti and (R)-BINOL followed by azeotrope, which serves as a moisture-tolerable enantioselective catalyst for the glyoxylate-ene reaction and shows an asymmetric amplification therein.

In another reaction Imma et al. [*Synlett,* 12, **1996,** 1229] used for an asymmetric catalytic hydrosilylation of ketones with HSi(OET)₃ (R)-BINOL-Ti(O-I-Pr)₂ as a precatalyst and observed levels of enantioselectivity up to 55 % and an "enantioselective autoinduction" of the reaction.

Zhang et al. [*Tetrahedron Asymmetry,* 8, **1997,** 585] described an enantioselective addition of diethylzinc to aldehydes by using a catalyst prepared in situ by mixing titanium tetraisopropoxide with S- or R-BINOL.

The asymmetric catalytic alkylation of aldehydes with diethylzinc using (R)-BINOL-Ti(O-I-Pr)₂ complex as an asymmetric precatalyst is disclosed by Mori and Nakai [*Tetrahedron Lett.,* 38, **1997,** 6233] in order to afford the corresponding secondary alcohols.

However, all these systems are interested solely in the abilities of the catalyst and not in the recovery and reuse of the ligand/catalyst.

Despite the relatively short period these BINOL/Ti systems have been studied, a number of groups have worked on recovering the ligand/catalyst. These approaches have included the use of fluorous biphasic conditions [Tian, Y., Yang, Q. C., Mak, T. C. W., Chan, K. S., *Tetrahedron,* 58, **2002**, 3951; Nakamura, Y., Takeuchi, S., Ohgo, Y., Curran, D. P., *Tetrahedron Lett.,* 41, **2000**, 57]. The main problems with all of the attempted recycling systems are that they require extensive functionalisation of the binaphthyl backbone before separation is achievable and, in many cases, the activity and selectivity of the modified BINOL catalysts are reduced in comparison to the unmodified systems, making the techniques employed for catalyst recovery detrimental to the overall catalytic process.

### Aims of the Invention

In view of the above, and due to the expensive nature of the resolved BINOL, ligand recovery is the aim of the present invention. It is therefore an object of the present invention to present a process for the separation and recovery of said BINOL ligand/catalyst as an alternative to the methods of recovery which have failed. A further object of the invention is to provide both a new method for catalyzing an asymmetric reaction in high conversion rate and enantioselectivity and for recovering and re-using of the ligand/catalyst without falls in activity or specificity.

### Disclosure of the invention

The present invention relates to a light fluorous approach to improving the method for the recovery and recycling of the synthetically important BINOL/Ti catalytic system.

As a result of experiments a methodology is developed for the allylation of aldehydes. In special it has been found that tagging the BINOL moiety with fluorinated side-chains and using a fluorous reverse phase silica enables the separation of products from catalyst.

The present invention further includes a method to regenerate the catalytic system from a new Sn(Bu)₃-BINOL polymer.

A further object of the present invention is to illustrate that this system does not decrease catalytic activity/efficiency.

In summary, the present invention provides a route to enable the recycling of a BINOL/Ti catalyst and demonstrates that this can be isolated and reused without any decrease in activity.

### Comparison of the two (R)-BINOL species

An initial study was performed on the addition of allyltri-n-butyltin to benzaldehyde (allyltri-n-butyltin is a common reagent for the addition of allyl groups to organic substrates and is, therefore, cheap and easily obtained) using both (R)-BINOL an (R)-Rf-BINOL in order to ascertain whether conversions and enantiomeric excesses (ees) would be effected by the presence of perfluoro-alkyl groups (Rf = C6F13). The reaction scheme is as follows:

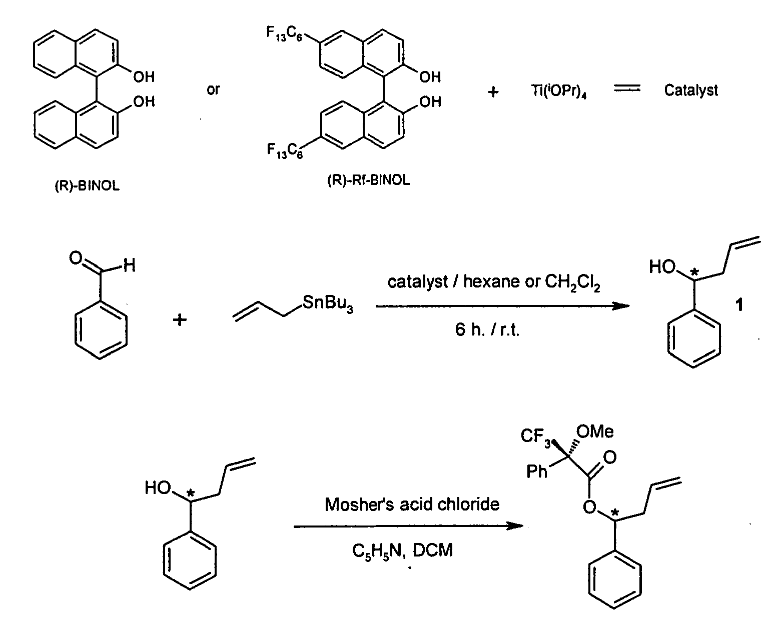

The progress of the reaction was determined by the conversion into the Mosher's acid ester followed by GC analysis.

The results after three separate reaction runs for (R)-BINOL and (R)-Rf-BINOL are summarized in the following table:

| **Ligand** | **Conversion (%)**^{**a**} | **ee (%)**^{**b**} |
|---|---|---|
| (R)-BINOL^{c} | 90 | 82 |
| (R)-BINOL^{c} | 90 | 78 |
| (R)-BINOL^{c} | 88 | 78 |
| (R)-Rf-BINOL^{d} | 86 | 74 |
| (R)-Rf-BINOL^{d} | 90 | 78 |
| (R)-Rf-BINOL^{d} | 88 | 74 |

| | | |
|---|---|---|
| ^{a}By ¹H NMR ^{b}By GC of Mosher's acid ester ^{c}In DCM, O°C, t=6 h ^{d}In hexane, O°C, t=6 h | | |

The results show that the inclusion of perfluoro-alkyl chains has no detrimental effect on the modified ligand. Also, the reaction is highly reproducible, with similar product conversions and enantiopurities observed after three separate runs.

### Separation of the Ligand from the Product (4-phenyl-1-buten-4-ol 1)

After each catalytic run the reaction mixture was concentrated *in vacuo.* After concentration of a reaction using (R)-BINOL crystals were noted to form. After NMR, MS and X-ray crystal analysis these were shown to be of a previously unreported Sn(Bu)₃-BINOL polymer. The tin-containing residue binds to the oxygens of BINOL forming a polymeric chain, and this is a very interesting structure because it suggests that the material to be isolated at the end of the reaction and reused may not be a titanium-containing species at all. Also, formation of this Sn-BINOL polymer clearly changes the separation dynamics of the mixture, suggesting that a non-polar solid separation media, such as fluorous reverse phase silica gel, should be much more appropriate for ligand retention.

Next, a standard catalytic addition was carried out using (R)-Rf-BINOL as the ligand. After concentration *in vacuo* the crude mixture was added to the top of a short column of fluorous reverse phase silica gel and eluted with acetonitrile.

Solvent was removed from the isolated material to yield a yellow oil which was shown to be 4-phenyl-1-buten-4-ol 1 by ¹H NMR, and there was no evidence of (R)-Rf-BINOL. The product conversion was determined to be 89% by ¹H NMR analysis and the ee to be 70% by ¹H NMR analysis of the Mosher's acid ester of the product. After removing the Sn-Rf-BINOL polymeric material from the column using diethyl ether, it was attempted to use this material in a standard catalytic run. Unfortunately, there was no evidence of reaction and only starting material was observed. This indicates that the polymeric species is not catalytically active. It can be envisaged that retention of the material on the column of fluorous reverse phase silica gel is due to the moiety's highly hydrophobic nature and subsequent favourable non-polar Van der Waals interactions with the solid support. This allows the relatively polar acetonitrile solvent to wash off the product from the column while leaving behind the non-polar Rf-BINOL-containing species and any free Rf-BINOL. Diethyl ether, which is a very fluorophilic solvent, can then be used to recover the RF-BINOL species and it was shown that the free (R)-Rf-BINOL can be recovered by washing this species, dissolved in hexane, with 4M hydrochloric acid.

Recovered (R)-Rf-BINOL was then used in three further catalytic runs using the same catalysis, separation and recovery methodology. The results are as follows:

| **Run** | **Conversion (%)**^{**a**} | **ee (%)**^{**b**} |
|---|---|---|
| 1 | 85 | 66 |
| 2 ^{c} | 85 | 63 |
| 3 ^{c} | 82 | 58 |
| 4 ^{c} | 78 | 58 |

| | | |
|---|---|---|
| ^{a}By ¹H NMR ^{b}By GC of Mosher's acid ester ^{c}Using ligand from previous run | | |

These data show that similar product conversions are achieved after each run, with a slight fall after the third ligand reuse. This is most likely due to mechanical losses of the ligand after each recycle rather than any deterioration of the ligand itself. The fall in product ee is also slight, and is probably due to a small amount of ligand racemisation. The conversions to product using this methodology are better than those observed previously and, unlike previously, the data shows the ligand can be successfully recycled after use in catalysis.

Several supports were investigated and results have indicated that fluorous reverse phase silica gel is required for successful separation and recovery of the (R)-Rf-BINOL ligand.

For the Rf-BINOL system the use of acetonitrile as eluant allows the ligand and product to be successfully separated using fluorous reverse phase silica gel. Then, with elution using diethyl ether followed by an acid wash, free (R)-Rf-BINOL can be recovered and reused in further catalysis without loss of activity or selectivity. This same outcome cannot be achieved if silica gel, C₈ reverse phase silica gel or powdered Teflon are used as the solid support, with leaching of the ligand-based species and contamination of the product occurring in all cases.

Preferably, Rf groups can be C4F9 to C12F25 and substitution of the BINOI can be at the 3, 4 and 6 positions..

The scope of this methology is wide and in principle can be applied to any system using BINOL (e.g. alkylation of benzaldehyde using diethylzinc). In general any Lewis acid-catalysed reactions will work.

Addition of the allyl group and subsequent separation is performed as described above.

### Industrial applicability

The light fluorous approach of the present invention enables the expensive, industrially useful, BINOL/Ti asymmetric catalytic system to be recovered and recycled. This will enable the costs of many synthetically common reactions to be reduced.

Now, the present invention will be described in further detail with reference to Examples. However, it should be understood that the present invention is not limited to such specific examples within the scope of the subject matter of the present invention.

### Examples

### General Catalysis Procedure for the Addition of Allyltri-n-butyltin to Benzaldehyde

To a solution of Ti(O^{*i*}Pr)₄ (0.3 ml in 20 ml hexane, 2 ml, 0.1 mmol), is added the BINOL ligand (0.2 mmol) and the mixture is stirred for one hour. Then the mixture is cooled to 0 °C and benzaldehyde (0.1 ml, 1 mmol) is added. The mix is stirred for 10 minutes and then allyltri-n-butyltin is added (0.34 ml, 1.1 mmol) and the reaction mixture is held at 0 °C for six hours.

The reaction is quenched with saturated NaHCO₃ solution (5 ml), washed with 1M hydrochloric acid (10 ml) and the mixture is filtered, dried (MgSO₄), filtered once more and the solvent removed *in vacuo* to yield the product as a colourless oil contaminated with ligand.

### General Procedure for the Separation of BINOL and 4-phenyl-1-buten-4-ol

A standard reaction mixture is concentrated in vacuo and the residue is placed onto the top of a column of silica gel, FRP silica gel, C₈-reverse phase silica gel or powdered PTFE 3 cm long and 1 cm in diameter. Acetonitrile is then used as elutant to recover the product. Diethyl ether is used as the second elutant to recover any ligand not eluted with the acetonitrile phase. Only when FRP silica gel and (*R*)-Rf-BINOL are used complete separation of the ligand and product is possible. (R)-Rf-BINOL is used in three further catalytic runs following the same catalysis procedure using the recovered ligand. The general separation procedure is then used to recover the ligand and separate the 4-phenyl-1-buten-4-ol product. After each run, the product is washed with 6M hydrochloric acid and the Sn and Ti levels are determined by ICP analysis of the wash. Figures in brackets indicate percentage of metal added at the outset that is present in the product. After separation of the ligand on FRP silica gel, the ether fractions are combined, washed with 4M hydrochloric acid, dried with MgSO₄ and the solvent is removed to yield (R)-Rf-BINOL. Run 1: 85 % conversion, 66 % ee. Sn: 1.24 ppm, (0.02 %) Ti: 2.21 ppm, (0.88 %); Run 2: 85 % conversion, 63 % ee. Sn: 0.75 ppm, (0.01 %) Ti: 1.99 ppm, (0.83 %); Run 3: 82 % conversion, 58 % ee. Sn: 2.73 ppm, (0.04 %) Ti: 2.86 ppm, (1.19 %); Run 4: 78 % conversion, 58 % ee. Sn: 4.40 ppm, (0.07 %) Ti: 4.65 ppm, (1.93 %).

### Asymmetric Addition of Allyltri-n-butyltin Using (R)-BINOL

The general catalysis procedure is followed using (R)-BINOL (57 mg, 0.2 mmol) as ligand. The product is collected as a colourless oil contaminated with ligand. m/z (ES-) 149 [MH]⁻ (23 %). δ_{H} 2.32 (1H, bs, OH), 2.54 (2H, um, CH₂), 4.74 (1 H, dd, ³*J*_{HH} 5.8 Hz, ³*J*_{HH} 7.0 Hz, CH), 5.15 (1H, um, =CH₂), 5.20 (1H, dum, ³*J*_{HH} 9.0 Hz, =CH₂), 5.84 (1 H, um, HC=), 7.24 - 7.39 (5H, um, PhH).

### Asymmetric Addition of Allyltri-n-butyltin Using (R)-Rf-BINOL

The general catalysis procedure is followed using (*R*)-Rf-BINOL (184 mg, 0.2 mmol) as ligand. The product is collected as a colourless oil. m/z (ES-) 149 [MH]⁻ (26 %). δ_{H} 2.32 (1 H, bs, OH), 2.54 (2H, um, CH₂), 4.74 (1H, dd, ³*J*_{HH} 5.8 Hz, ³*J*_{HH} 7.0 Hz, CH), 5.15 (1H, um, =CH₂), 5.20 (1H, dum, ³*J*_{HH} 9.0 Hz, =CH₂), 5.84 (1H, um, HC=), 7.24 - 7.39 (5H, um, PhH).

### Procedure for the Determination of 4-Phenyl-1-buten-4-ol Product ee

Mosher's acid chloride (250 mg in 10 ml DCM, 990 µM, 2 ml, 0.2 mmol) and pyridine (0.2 ml, 2.5 mmol) are added to a flame-dried flask under nitrogen. To this mixture, 4-phenyl-1-buten-4-ol (29 µl, 0.2 mmol) is added and the reaction mixture is stirred for 30 minutes.
1M hydrochloric acid is added (3 ml) and the biphase is transferred to a separating funnel. The organic phase is separated and washed with NaHCO₃ (10 ml) and water (10 ml), dried (MgSO₄), filtered and the solvent removed *in vacuo* to yield a colourless oil which is analysed by chiral GC for diastereomeric content (CYDEX-B, 180 °C for 20 min. Injector: 220 °C, detector: 250 °C. Flow rate: 2 ml/min (*R*)-4-phenyl-1-buten-4-ol, Mosher's acid ester Rₜ 8.81 min, (*S*)-4-phenyl-1-buten-4-ol, Mosher's acid ester, Rₜ 13.00 min).

## Claims

1. A process for the preparation of asymmetrically substituted compounds using a light fluorous approach, in which a fluoroalkyl tagged 1,1'-Bi-2-Naphthol [(R)-BiNOL] derivative and titanium tetraisopropoxide catalytic system is used,**characterized in that** the catalytic system is recovered after the reaction by
a) adding the crude mixture on the top of a reverse phase chromatography column
b) eluting the reaction product,
c) isolating the formed Sn-Rf-BINOL polymeric material, which is formed during the asymmetric catalytic reaction,
d) recovering free (R)-Rf-BINOL from the solution received in step c) with an acidified non-polar solvent and
e) reusing the recovered (R)-Rf-BINOL in an asymmetric catalytic reaction.

2. Process according to claim 1, **characterized in that** the chromatography column is packed with a suitable solid support selected from the group silica gel, FRP silica gel, C₈ reverse phase silica gel, and powdered poly(tetrafluoroethene.

3. Process according to claim 1, **characterized in that** the chromatography column is packed with a fluorous reverse phase silica gel.

4. Process according to claim 1, **characterized in that** the formed Sn-Rf-BINOL polymeric material is isolated from the solid support by the use of an aprotic polar solvent.

5. Process according to claim 4, **characterized in that** the formed Sn-Rf-BINOL polymeric material is isolated from the solid support by the use of diethyl ether.

6. Process according to claim 1, **characterized in** washing off the non-polar Rf-BINOL species and any free (R)-Rf-BINOL with a fluorophilic solvent selected from the group diethyl ether, tetrahydrofuran, acetone and perfluorinated solvents.

7. Process according to claim 1, **characterized in that** for recovering free (R)-Rf-BINOL from the solution received in step c) a non-polar solvent is acidified with a weakly acidic solution.

8. Process according to claim 1, **characterized in that** for recovering free (R)-Rf-BINOL from the solution received in step c) a non-polar solvent, such as hexane, is acidified with a weakly acidic solution obtained from the mineral acid hydrochloric acid.

9. Process according to the claims 1 - 8 for the production of asymmetric materials.
